Europäisches Patentamt

European Patent Office

Office européen des brevets

(11) Publication number: **0 468 220 A2**

## EUROPEAN PATENT APPLICATION

(21) Application number: **91110631.8**

(22) Date of filing: **27.06.91**

(51) Int. Cl.⁵: **C12N 15/52**, C12N 15/76,
C12P 29/00, C12N 1/21,
//C12N15/74,C12Q1/68,
(C12N1/21,C12R1:465),
(C12N15/52,C12R1:485)

(30) Priority: **26.07.90 US 558039**

(43) Date of publication of application:
**29.01.92 Bulletin 92/05**

(84) Designated Contracting States:
**AT BE CH DE DK ES FR GB GR IT LI LU NL SE**

(71) Applicant: **AMERICAN CYANAMID COMPANY**
**1937 West Main Street P.O. Box 60**
**Stamford Connecticut 06904-0060(US)**

(72) Inventor: **Lotvin, Jason A.**
**985 Roosevelt Avenue**
**Union, New Jersey 07083(US)**
Inventor: **Ryan, Michael J.**
**34 Northwood Drive**
**West Milford, New Jersey 07483(US)**
Inventor: **Strathy, Nancy**
**16 Edgebrook Lane**
**Monsey, New York 10952(US)**

(74) Representative: **Wächtershäuser, Günter, Dr.**
**Tal 29**
**W-8000 München 2(DE)**

(54) **Cloning of the biosynthetic pathway genes for chlortetracycline production from streptomyces aureofaciens & their expression in steptomyces lividans.**

(57) The present invention relates to the cloning, isolation and DNA (isolated fragment from Streptomyces aureofaciens) which directs the synthesis of tetracycline and chlortetracycline, in a heterologous host such as Streptomyces lividans. The claimed plasmids are isolated by screening a cosmid library, constructed in Escherichia coli, for the expression of tetracycline resistance in S. lividans.

EP 0 468 220 A2

## BACKGROUND OF THE INVENTION

The present invention relates to the application of molecular genetic techniques for cloning of the gene cluster (DNA) encoding the entire pathway for chlortetracycline, and thus also tetracycline biosynthesis from S. aureofaciens and its expression in the heterologous host S. lividans. The isolated biosynthetic gene cluster (DNA) serves as a substrate for bioengineering enhanced fermentation yields as well as novel antibiotic structures.

The antibiotic chlortetracycline and its derivative compounds (e.g. tetracycline, demethylchlortetracycline, demethyltetracycline) are produced commercially in submerged fermentation by Streptomyces aureofaciens (Dugar , 1948). More than 30 years of industrial manipulation of this microorganism has resulted in the development of sophisticated fermentation techniques and media formulations that have allowed significant improvements in fermentation yield (Goodman, 1985). These advances in yield improvement have also been aided by the isolation of mutants of S. aureofaciens with increased ability to produce antibiotic (Veselova, 1969). These high-producing strains have largely been isolated by the process of mutagenesis, followed by random screening for improved yield. The same techniques allowed the isolation of mutants blocked in antibiotic biosynthesis which were critical tools for the elucidation of the biosynthetic sequence for chlortetracycline formation (Mccormick, 1968). Despite these accomplishments, an understanding of the genetic regulation of chlortetracycline biosynthesis was not completely realized. Recent developments in the field of Streptomyces genetics have created the opportunity to study molecular genetics of organisms producing industrially important metabolites.

The demonstration of recombination of chromosomal markers by the fusion and subsequent regeneration of Streptomyces protoplasts (Hopwood, et al, 1978 and Baltz, et al, 1981) was a pivotal event in the genetics of the actinomycetes. Whereas prior to the development of techniques for protoplast fusion, genetic crosses could only be reliably performed in a few species with demonstrated conjugal systems (Hopwood, 1967), now genetic analysis can be performed in any species which can be protoplasted and regenerated. More importantly, protoplasts later proved to be an ideal substrate for transformation by plasmid DNA, thus creating the opportunity to do recombinant DNA experiments in these organisms (Bibb, et al, 1978). The isolation of genes for several antibiotic resistances such as, thiostrepton, viomycin and neomycin, allowed the construction of readily selectable cloning vectors from indigenous Streptomyces plasmids (Thompson, et al, 1982).

One of the first antibiotic biosynthetic genes to be cloned was the O-methyltransferase involved in the formation of the antibiotic pigment undecyl-prodigiosin (UDP.) (Feitelson, et al, 1980). The gene was identified by its ability to complement a known mutation in the UDP biosynthetic pathway. Other techniques employed in these early efforts to isolate biosynthetic genes included mutational cloning using phage OC31 for methylenomycin (Chater, et al, 1983) a sib selection of recombinant clones using in vitro enzyme assays for the actinomycin phenoxazinone synthetase (Jones, et al, 1984) and sulphonamide resistance conferred by the p-aminobenzoic acid synthetase involved in candicidin production (Gil et al, 1983). Bialaphos biosynthetic genes were identified via complementation of blocked mutants (Murakami et al, 1986).

Genes involved in actinorhodin biosynthesis were cloned by complementation of biosynthetically blocked mutants of Streptomyces coelicolor - (Malpartida et al, 1984). In this last case, two overlapping clones complementing distinct classes of mutants were combined on a single plasmid which was shown to confer the ability to synthesize actinorhodin when introduced into a heterologous Streptomyces parvulus host.

Another important series of observations was that genes for antibiotic biosynthesis were physically linked to the resistance determinant(s) for that same antibiotic in the producing organism. Thus, a DNA fragment from Streptomyces griseus conferring streptomycin resistance was shown to be contiguous with DNA that complemented biosynthetic blocks (Distler et al, 1985). The same situation was seen in Streptomyces fradiae where biosynthetic genes had been identified by probing a cosmid library for homology to a mixed-base oligonucleotide constructed to represent the DNA sequence for the amino-terminus of the final enzyme in the tylosin biosynthetic pathway (Fishman et al, 1989). A previously cloned tylosin resistance gene (tlrB) was shown to be contained within this region of DNA, which complemented nine classes of blocked mutants (Baltz et al 1988). In the cases of puromycin (Var2 et al, 1988) and tetracenomycin, (Motamedi, et al, 1987), a primary selection for expression of the antibiotic resistance gene in the heterologous host Streptomyces lividans allowed subsequent identification of antibiotic biosynthetic genes located on the same cloned DNA fragment.

The use of nucleic acid probes has aided the isolation of biosynthetic genes. This approach relies on the existence of a pre-existing body of information concerning the pathway or prior cloning having been performed. Thus, in the case of tylosin above, a probe was constructed using information from a partial amino acid sequence of a biosynthetic enzyme (Fishman, et al, 1987). Similarly, the gene for isopenicillin N synthetase was cloned from Streptomyces clavuligerus by identifying a clone hybridizing to an oligonucleotide probe constructed with a knowledge of the N-terminal amino acid sequence of the enzyme (Leskiw, 1988). Genes involved in the biosynthesis of erythromycin were identified by probing a cosmid library with a previously cloned erythromycin resistance gene (Stanzak, 1986). Similarly, genes involved in the biosynthesis of oxytetracycline have been identified by hybridization to both a previously cloned resistance determinant (Butler et al, 1989) and an oligonucleotide synthesized to represent the DNA sequence corresponding to the partially elucidated amino acid sequence of the biosynthetic enzyme anhydrotetracycline oxygenase (Binnie et al, 1989). The use of heterologous actl and actlll probes allowed the identification of genes involved in anthracycline biosynthesis in Streptomyces peucetius - (Stutzman-Engwall et al, 1989).

The use of these techniques individually or in combination has allowed the isolation or assembly of entire biosynthetic pathways, and in some instances, their expression in a heterologous host. The entire biosynthetic cluster for bialaphos was cloned by a combination of selections for complementing activities and heterologous expression of bialaphos resistance (Murakami et al, 1986). Although a note was made concerning a successful isolation of the entire pathway in a single step in Streptomyces lividans by selecting for bialaphos resistance, no mention is made concerning expression of the biosynthetic genes. The assembly of the actinorhodin cluster from complementing clones and its expression in Streptomyces parvulus was discussed (Malpartida et al, 1984) hereinabove.

A bifunctional cosmid clone which hybridized to a homologously derived erythromycin resistance determinant was isolated from a Saccharopolyspora erythrea library and shown to direct the synthesis of erythromycin when transferred to Streptomyces lividans (Stanzak et al, 1986). An E. coli cosmid clone that showed hybridization to both an oxytetracycline resistance gene probe and biosynthetic gene probe (for anhydrotetracycline oxygenase) allowed the isolation of the oxytetracycline biosynthetic cluster from Streptomyces rimosus (Binnie et al, 1989). Subsequent subcloning into a Streptomyces plasmid vector allowed production of oxytetracycline in Streptomyces lividans.

Two overlapping clones from the tetracenomycin producer were identified by complementation of blocked mutants of Streptomyces glaucescens and ability to confer tetracenomycin resistance in S. lividans (Motamedi et al, 1987). When both were separately resident in S. lividans and co-fermented, or when they were co-resident in the same S. lividans host, tetracenomycin was produced. Bifunctional clones isolated from an E. coli library of Streptomyces peucetius DNA by hybridization to actl and actlll probes of S. coelicolor were shown to direct the synthesis of pigmented antibiotic when introduced into S. lividans - (Stutzman-Engwall, 1989).

Additionally, the isolation of the biosynthetic pathway for cepthamycin C production has occurred (Chen et al, 1988). In this case, random clones in S. lividans were individually screened for cephamycin C production using an agar plug fermentation method. One transformant of S. lividans out of 30,000 screened, was shown to be producing cephamycin C.

The present invention is the first instance wherein the DNA genes related to the biosynthetic pathway for producing tetracycline and chlortetracycline are isolated and utilized.

BRIEF DESCRIPTION OF THE DRAWINGS

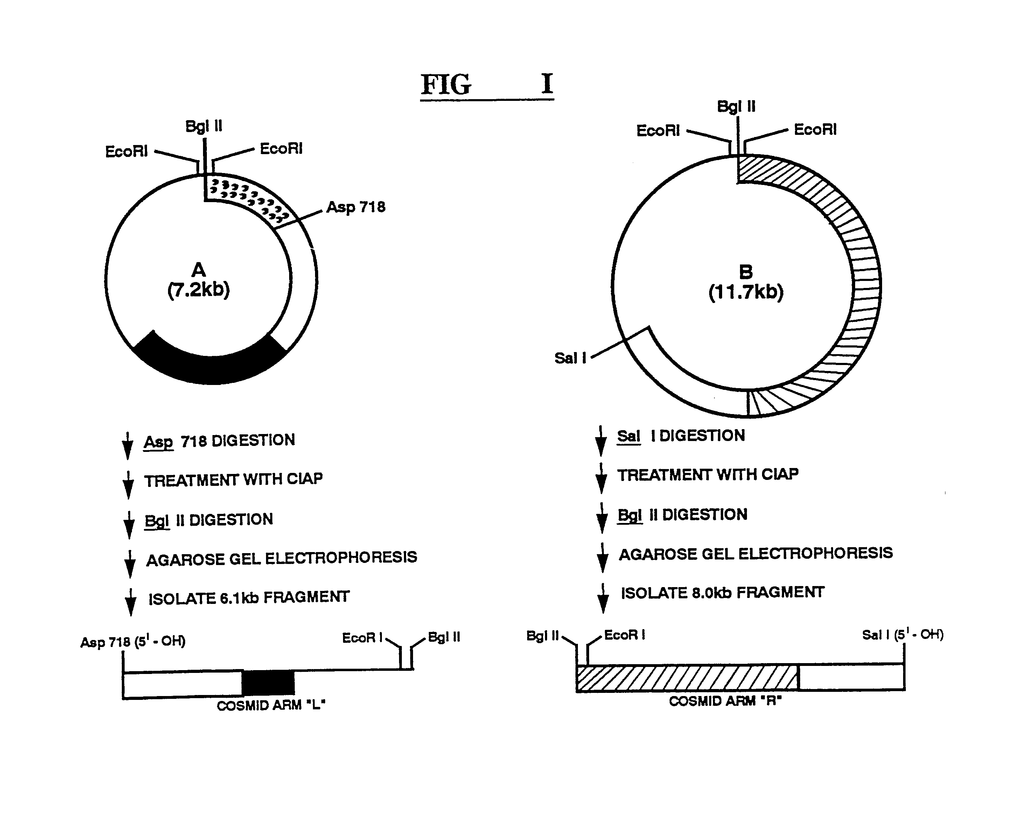

Figure 1: Structure of the components of the bifunctional cosmid vector and mehtod for generating cosmid arms. Cosmid vector arms L and R are generated from plasmids A and B, respectively, as shown in the figure and as detaled in Example 3. Single lines represent Escherichia coli replicon portions of the constructs. In plasmid A the E. coli portion is derived from the 3.7kb EcoRI-Sall fragment of pBR322 (Sutcliffe, 1979). Plasmid B contains a 5.9kb EcoRI-Sall fragment from SCP2* [striped] that provides for replication function in the actinomycetes (Larson et al, 1986). Three tandem cohesive end sites derived from a 700bp BglII-BstEII cos-containing fragment of pHC79 (Hohn et al, 1980) are provided on both plasmids [open]. The thiostrepton-resistance gene [darkened] present in plasmid A is derived from a l.lkb BclI fragment recovered from plJ702 (Katz et al, 1983). A 1.1kb spacer region in plasmid A [stippled] is derived from a SacI fragment of bacteriophage λ (Sanger et al, 1982).

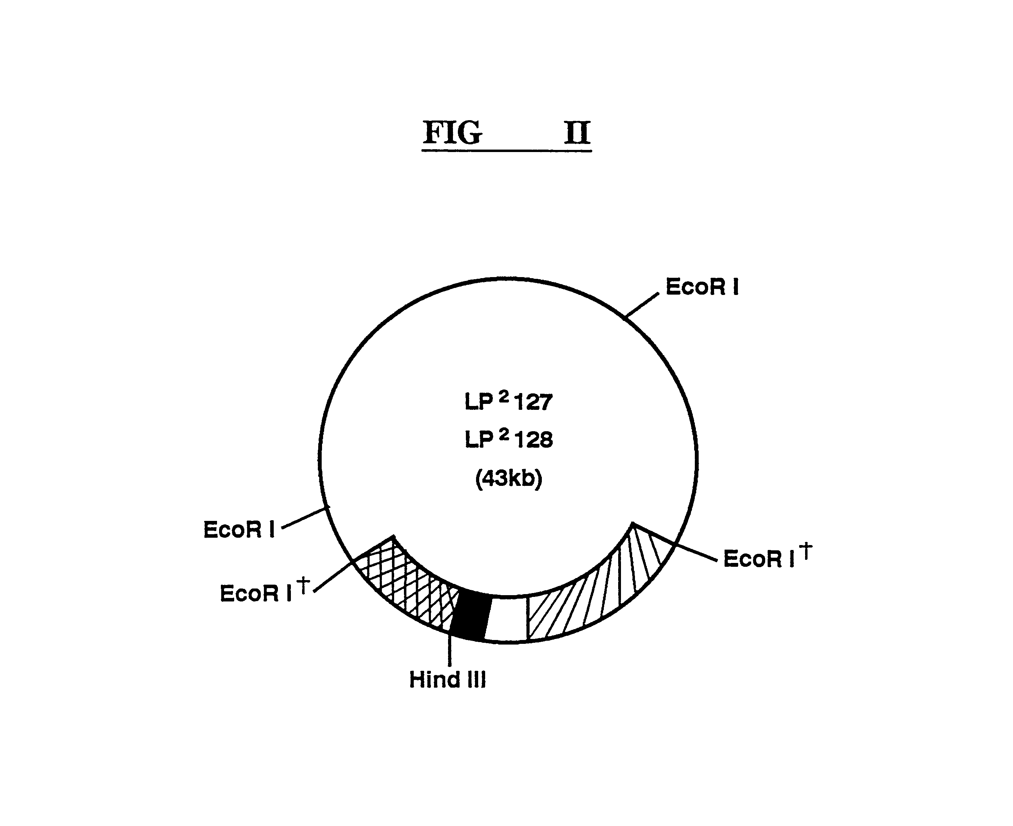

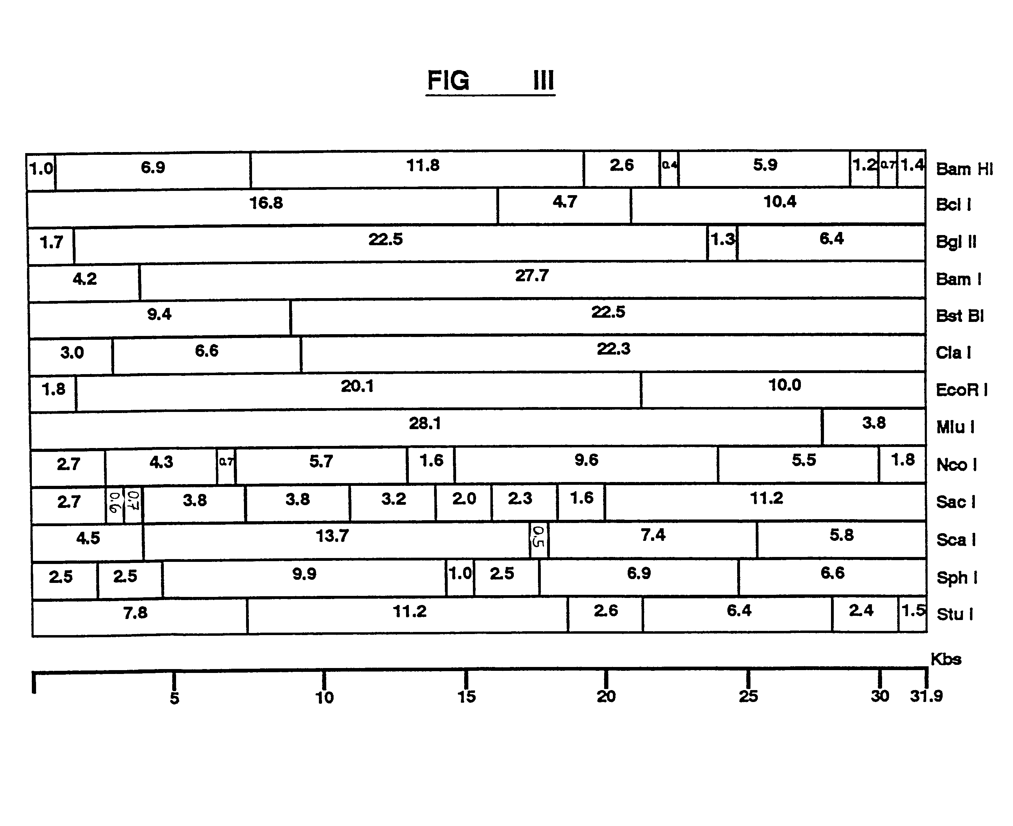

Figure 2: Physical map for LP$^2$127 and LP$^2$128. Both plasmids show equivalent structures by restriction mapping; therefore, a single stucture, representative of both, is shown here and in Figure 3. The vector portion is represented by double line; the TC/CTC biosynthetic region is shown as a single line. The DNA cloned from S. aureofaciens is 31.9kb; the vector is 11.1kb. The vector regions denoted are plBI-24 [hatched], thiostrepton-resistance [striped]. The two EcoRI sites marked with a (+) are vector-derived and flank the Sau3A-Bg1II junction which demarcates vector and S. aureofaciens DNA.

Figure 3: Restriction endonuclease map for S.

aureofaciens DNA cloned in LP$^2$127 and LP$^2$128. The 31.9kb of DNA cloned in LP$^2$127 and LP$^2$128 is shown in linear form. The map is drawn so as to include the EcoRI sites derived from the vector as the start and finish positions at the left and right. The sizes of restriction fragments are presented in kilobase pairs and are accurate to within the normal resolution limits of agarose gel electrophoretic analyses (~500bp).

SUMMARY OF THE INVENTION

The present invention relates to the cloning of the entire biosynthetic pathway for the formation of tetracycline and chlortetracycline from Streptomyces aureofaciens and its expression in the heterologous host Streptomyces lividans. Therefore, the present invention relates to the isolated DNA gene(s) (cluster) coding for the biosynthetic pathway for producing these antibiotics, chlortetracycline and tetracycline and to DNA which under stringent hybridization procedures, known to those in the art, hybridizes to DNA isolated gene clusters encoding for the pathway for the formulation of chlortetracycline and tetracycline. Further, the present invention relates to the use of these genes to increase yields of these antibiotics and in methods for screening for producing new analogues to tetracycline and chlortetracycline.

In order to proceed with the isolation of the biosynthetic genes, a screen of a recombinant S. lividans library for a clone expressing tetracycline-resistance is employed. The S. aureofaciens DNA inserts in the recombinant cosmids which comprise the library are necessarily large since the constraints of the in vitro lambda packaging system employed demands cosmid molecules with DNA inserts of 25-40Kb in order to yield a viable transducing phage particle. When tetracycline resistant clones are selected from amongst this population of S. aureofaciens genomic clones, a limited subset of cosmid clones is selected. Many or all of these are expected to contain antibiotic biosynthetic genes linked to the selected tetracycline resistance gene. Amongst those that are sufficiently large and correctly positioned is a subset encompassing the entire biosynthetic pathway. Thus, the cloning of genes, and in fact all of the genes, for tetracycline and chlortetracycline formation is possible without any preexisting knowledge of the structure or sequence of the region. Although reports have been published concerning the cloning of a tetracycline-resistance determinant (Reynes et al, 1988) and a bromoperoxidase (Vam Pee, 1988) from Streptomyces aureofaciens, these studies are in no way extended toward the isolation of chlortetracycline biosynthetic genes or the entire gene cluster.

The more detailed description of the present invention is provided hereinbelow through examples which are illustrative of the invention and not limitative of it.

DETAILED DESCRIPTION OF THE INVENTION

The method used for isolation of the DNA involves lysozyme digestion of cells in an osmotic buffer, followed by gentle lysis, protein extraction and enrichment for, and concentration of, high molecular weight DNA. Although the method described is efficient, those skilled in the art will recognize that a variety of alternative procedures may be employed, such as those described by Hopwood et al, 1985.

The source of total DNA used in the example is Streptomyces aureofaciens ATCC 13899 but the invention is in no way limited to this particular source. A variety of other Streptomyces aureofaciens strains producing antibiotics of the tetracycline class could be used in the present invention with equal success. These S. aureofaciens strains include mutant strains and alternative wild-type isolates producing chlortetracycline, tetracycline, 6-demethylchlortetracycline, 6-demethyltetracycline, 7-chloro-5a,11a dehydrotetracycline, 2-decarboxamido-2-acetyltetracycline and other members of the tetracycline family of compounds. The present invention relates also to the cloning of chlortetracycline, tetracycline and tetracycline-related compounds from other organisms producing such compounds, which include, but are not limited to Streptomyces rimosus, S. avellaneus, S. lusitanus, S. viridifaciens, S. psammoticus, Actinomadura brunnea, and Dactylosporangium vesca.

A partial digestion of S. aureofaciens DNA with restriction endonuclease Sau3A to generate large DNA fragments in the desired 35-kilobase size range with ends homologous to those of the arms of bifunctional cosmid vector is employed. In this case, an empirical determination of the optimal digestion conditions is obtained by conducting a series of digestions and analyzing a sample of the end products by agarose gel electrophoresis. Those skilled in the art recognize alternative library construction and recovery methods for cloned DNA of interest. The use of E. coli, as well as the size selection imposed by lambda packaging, of the example is not limitative of the present invention since the use of other vectors is useful as well. Those skilled in the art recognize that monofunctional Streptomyces vectors, such as pIJ922 (Lydiate et al, 1985) can be employed in the present invention, with the attached proviso that library construction and recombinant plasmid recovery is conducted within the actinomycetes.

The steps that follow in the examples involve in vitro packaging of the ligation products of cosmid arms and size fractionated DNA, transduction to E. coli X2819T, collection of the population of transductants and isolation of DNA from them to give a cosmid library. The methods used are described, but the invention is not limited by those described in the example. Alternative methods could be employed to the same end with no untoward consequences. Thus, alternative protocols for ligation and in vitro packaging may be employed, as well as alternative recombination-deficient (recA) E. coli hosts, library amplification procedures (e.g. selective broth growth) and plasmid preparation procedures, all of which have been published in the scientific literature (Maniatis et al, l982).

Subsequent steps in the examples describe introduction of the pooled cosmid DNA preparation into Streptomyces lividans, creation of a cell library and subsequent screening of such a library for transformants of S. lividans exhibiting resistance to l00 μg of tetracycline/mL. Though more laborious, transformants could be directly screened for tetracycline-resistance by replica plating. Alternative levels of tetracycline could be used for screening as dictated by the innate resistance exhibited by the host or source organisms. Other tetracycline-sensitive, non-restricting hosts, such as Streptomyces griseofuscus, could be substituted for S. lividans.

Next, recovery of recombinant plasmid by isolating plasmid DNA from the tetracycline-resistant S. lividans followed by in vitro packaging of said DNA and transduction into E. coli is obtained. Plasmid DNA isolated from such transductants is structurally characterized by restriction enzyme mapping analysis; and the two plasmids isolated in the example, $LP^2127$ and $LP^2128$, are shown to possess equivalent structures. Those skilled in the art will recognize that similar DNA regions cloned from alternative organisms could show polymorphism in the arrangement of restriction sites, but that a sufficiently large DNA fragment conferring tetracycline-resistance would be expected to confer the properties described hereinbelow.

The plasmid-borne nature of the tetracycline resistance is confirmed by demonstrating that thiostrepton-resistant transformants of S. lividans obtained with $LP^2127$ and $LP^2128$ are also tetracycline resistant. The elaboration of tetracycline-like antibiotic is demonstrated by the production on agar by the aforementioned thiostrepton and tetracycline resistant S. lividans of antibiotic activity effective against E. coli but less so against a tetracycline-resistant E. coli.

Finally, it is demonstrated that the synthesis of tetracyclines is directed by $LP^2127$ in the heterologous host Streptomyces lividans. This is accomplished in both agar and broth fermentation. Both the originally isolated tetracycline resistant S. lividans and a $LP^2l27$ transformant of S. lividans produce tetracycline and chlortetracycline under conditions where the same products are isolated from the DNA source organism Streptomyces aureofaciens ATCC l3899. On the other hand, a S. lividans transformant containing only plasmid vector with no inserted DNA shows no antibiotic production.

The demonstration of production of tetracyclines by the heterologous host is not limited to the fermentation conditions or HPLC analytical systems described in the example, although these clearly allow efficient analysis. A large number of procedures for fermentation and analysis of tetracyclines have been described and can be substitued herein. Also, although Streptomyces lividans is used as the heterologous host in the examples, the heterologous expression of antibiotic biosynthetic genes is expected in a number of actinomycetes and other bacterial groups including, but not limited by Bacillus, Corynebacteria, Thermoactinomyces, so long as they are transformed with the relatively large plasmid constructions described here. Those that are transformed include such as Streptomyces griseofuscus and Streptomyces ambofaciens which are known to be relatively non-restricting.

EXAMPLE 1:PREPARATION OF STREPTOMYCES AUREOFACIENS TOTAL DNA

A lyophilized preparation of Streptomyces aureofaciens ATCC l3899 is suspended in 0.8 mL of lX synthetic salts solution (6 g of $Na_2HPO_4$/L, 3g of $KH_2PO_4$/L, 0.57g of sodium citrate/L) and plated onto Bennett's agar (l g of yeast extract/L, 2g of NZ-Amine A/L, l g of beef extract/L, 20 g of D-glucose/L, 20 g of Bacto-agar/L). After incubation at 28°C for two days, cells from a single plate are scraped into 5 mL of Tryptic Soy Broth (Difco) and sonicated briefly (~l0 seconds) with a Heat Systems Ultrasonics W200P sonicator equipped with a microtip. A seed culture is developed by inoculating 2mL of the sonicated suspension into 50 mL Tryptic Soy Broth (TSB), followed by incubation at 28°C, 200 rpm for 2 days. Five mL of seed culture is then inoculated to l00 ml TSB supplemented with 2% glycine and incubated at 28°C, 200 rpm for 48 hrs.

Cells are harvested by centrifugation at 9800Xg for 30 minutes. The cell pellet is washed with 200 mL P medium (l00 g of sucrose/L, 0.2 g of $K_2SO_4$/L, 2 mL of trace element solution/L which consists of 40 mg of $ZnCl_2$/L and l0 mg/L each $FeCl_3$.$6H_2O$, $CuCl_2$.$2H_2O$, $MnCl_2$.$4H_2O$, $Na_4B_2O_7$.$l0H_2O$ and $(NH_4)_6$ $MO_724$.$4H_2O$). The cell

pellet is frozen at -20°C then defrosted and suspended in 12 mL p<sup>+</sup> (P medium suppplemented to contain 25 mM TES, 25 mM CaCl$_2$, 10 mM MgCl$_2$, 03.7 mM KH$_2$PO$_4$) containing 10 mg/mL lysozyme (Sigman, 3X recrystallized). The cells are incubated at room temperature for 2 hours by which time protoplast formation is evident. An addition of 2.5 mg of Proteinase K (Boehringer-Mannheim is made and the mixture incubated at 37°C for 15 minutes. Lysis is achieved by adding 10 mL 0.2M EDTA pH8, 0.1M Tris pH 8 followed immediately by the addition of 2.4 mL 10% sodium lauryl sulfate (SDS). The viscous mixture is incubated at 50°C for 60 minutes with occasional gentle mixing.

Once lysis is complete, 20 mL of equilibrated phenol (50 g phenol + 6.5 mL of 100 mM NaCl, 10 mM Tris pH8, 1 mM EDTA pH8 + 0.05 g 8-hydroxyquinoline) is added, the preparation gently shaken and then spun in a table top centrifuge at 1500 X g for 30 minutes. The aqueous top layer is collected and re-extracted as above; the spent phenol from the first extraction is back-extracted with 20 mL 10 mM Tris pH7.4, 1 mM EDTA pH 8 (TE). The collected aqueous phases are then extracted with an equal volume of chloroform, spun as above and 10-mL portions distributed to separate test tubes. One mL of 3M ammonium acetate pH 5 is added to each and 10 mL of cold ethanol layered on top of the viscous solution. The DNA is gently spooled onto a glass rod, rinsed twice in cold ethanol and dissolved in 8 mL TE overnight at 4°C. An A$_{260}$ spectrophotometric reading is taken as an estimate of total nucleic acids present (predominantly DNA).

EXAMPLE 2: PARTIAL DIGESTION AND SIZE ENRICHMENT OF S. AUREOFACIENS DNA

A partial digestion condition that yields Sau3A digestion products of S. aureofaciens DNA in the range of 35 kilobases (Kb) is determined empirically. A series of reaction tubes containing -25 $\mu$g DNA contained in 300 $\mu$L of reaction buffer consisting of 100 mM NaCl, 10 mM Tris pH7.4 10mM MgCl$_2$ are prepared, and restriction endonuclease Sau3A (New England Biolabs) added to give final concentrations of 0.5, 0.1, 0.05, 0.01, 0.005 enzyme units/$\mu$g DNA. The reactions are incubated at 37°C for 60 minutes, then placed at 65°C for 20 minutes and finally removed to ice. Twenty $\mu$L is removed and loaded to 0.5% agarose gel for size comparison to fragments of known length (lambda DNA digested with HindIII, XhoI and undigested). The DNA in the remaining volume is precipitated by the sequential additions of 50 $\mu$L 3M ammonium acetate and 1 mL ethanol, followed by chilling at -20°C. The precipitated DNA is then pelleted by centrifugation at 8800Xg, redissolved in 300 $\mu$L 0.3M

ammonium acetate, similarly precipitated, pelleted, rinsed with ethanol, vacuum dried and the dried pellet finally dissolved in 100$\mu$L TE. An inspection of the ethidum bromide stained agarose gel which is electrophoresed overnight at 1 volt/cm, reveals that digestion with 0.05 units Sau3A/$\mu$g DNA gives digestion products largely in the desired 35Kb size range.

EXAMPLE 3: PREPARATION OF COSMID ARMS

The components of the bifunctional cosmid vector are retrieved from plasmids A and B shown in Figure I. Plasmid A contains pIBI24 which provides an origin of replication and an ampicillin resistance gene for replication and selection in E. coli. This plasmid also provides a thiostrepton-resistance gene for plasmid selection in the actinomycetes, as well as multiple cohesive end sites (cos) from bacteriophage lambda which serve as substrates for in vitro packaging. Plasmid B is designed to provide an SCP2* origin of replication for plasmid maintenance in the actinomycetes, and multiple cos sites.

Plasmid A is digested with Asp718 and then desphosphorylated with calf intestine alkaline phosphatase (CIAP). The DNA then is extracted with chlorpane and chloroform, precipitated with ethanol and vacuum dried. The DNA is then re-suspended and digested with BgIII. Plasmid B is digested with SalI and subsequently treated with CIAP. After chlorpane extraction, ethanol precipitation and vacuum drying, the DNA is resuspended and digested with BgIII.

The digestion reactions noted above are loaded to an agarose gel and electrophoresed overnight. A 6 Kb fragment from plasmid A and an 8.0 Kb fragment from plasmid B, which contain the functional regions described above, are isolated from the agarose gel by electroelution.

EXAMPLE 4: LIGATION OF COSMID ARMS TO SAU3A DIGESTED GENOMIC DNA AND IN VITRO PACKAGING

The Sau3A digested and size "inspected" genomic fragments of S. aureofaciens DNA are joined to cosmid arms via in vitro ligation. Four $\mu$L Sau3A digested S. aureofaciens DNA, corresponding to ~8 $\mu$g, are combined with 1 $\mu$g each of cosmid arms 1 and 2 in a 10 $\mu$L ligation mixture that contains 66mM Tris pH7.4, 10 mM MgCl$_2$, 1 mM ATP, 10 mM dithiothreitol and 40 units (cohesive end unit) T4 DNA ligase (New England Biolabs). The ligation mixture is incubated at 11°C for 18 hours then subjected to an in vitro packaging reaction by adding the entire 10 $\mu$L reaction to a Packagene<sup>R</sup> lambda DNA packaging system extract (Promega Biotec). After a 2 hour incubation at room temperature, 500 $\mu$L phage dilution buffer (PDB) (l00 mM NaCl, l0 mM TRIS-HCl pH 7.4, l0 mM MgSO$_4$) is added followed by the addition of 25 $\mu$L chloroform. The mixture is vortexed and stored at 4°C.

## EXAMPLE 5: TRANSDUCTION INTO ESCHERICHIA COLI AND PREPARATION OF A BIFUNCTIONAL COSMID LIBRARY

The phage preparation derived from the in vitro packaging reaction is transduced into Escherichia coli X2819T (R. Curtiss), with the objective of obtaining thousands of transductants from which a pooled plasmid DNA preparation, or bifunctional cosmid library, can be obtained. To this end, 0.3 mL of an overnight culture of X2819T is inoculated into l0 mL 20-10-5 (20 g of Tryptone/L, l0 g of yeast extract/L, 5 g of NaCl/L, 50 mg of thymidine/L) and incubated at 28°C, 2.5 hours. Four portions of 0.4 mL X2819T cells are then combined with 0.8 mL PDB and spun in a microfuge at full speed for 5 minutes. The pelleted cells are suspended in l00 $\mu$L PDB. Then 50 $\mu$L of phage preparation from in vitro packaging is added to each. Phage are absorbed to cells at 37°C, 25 minutes. Two mL of 20-10-5 are added to each mixture and the suspension incubated with shaking at 28°C for 2 hours. One-tenth mL aliquots are plated onto a total of 50 Petri plates containing 20-10-5 agar (20-10-5 broth and 20 g of Bacto agar/L) supplemented with l00 mg of ampicillin/L (sodium salt-Sigma). The plates are incubated at 28°C overnight and then left at room temperature for three days. A plate count of five representative plates reveals that a total of ~12,000 ampicillin-resistant colonies are obtained.

Each plate is flooded with 5 mL of solution consisting of 50 mM glucose, 25mM Tris-HCl pH 8, l0 mM EDTA pH 8 (GTE). The colonies are suspended with a sterile spreader and all eluates pooled to yield a cell suspension which is spun at 9800Xg for 5 minutes. The pelleted cells are resuspended in 72 mL-GTE. Then 8 mL of GTE containing 40 mg of lysozyme/mL is added. The lysozyme digestion is incubated at room temperature for 20 minutes. Then l60 mL of alkaline-SDS (8 g of NaOH/L, l0 g of SDS/L) is added which yields a viscous lysate after gentle mixing. After incubation on ice for 20 minutes, 80 mL of 5M potassium acetate is added, mixed, and incubated an additional 20 minutes on ice. The preparation is then spun at 9800Xg for 20 minutes, the supernatent collected and 200 mL of cold isopropanol added, mixed in and incubated on ice for l5 minutes, followed by centrifugation at 9800Xg for 20 minutes. The nucleic acid pellet is dissolved in 20 mL TE supplemented with l% sodium sarcosine.

Twenty-two grams of CsCl is added, and once dissolved, 2 mL of a solution of l0mg ethidium bromide/mL is added. The CsCl-ethidium bromide mixture is loaded into appropriate tubes and centrifuged in a Beckman 70.1Ti rotor at 55,000 rpm for l9 hours. The tubes are removed and plasmid band is recovered by syringe side puncture. Ethidium bromide is removed from the sample by extracting 4 times with equal volumes of butanol saturated with water. The aqueous solution is brought to 6 mL with TE; l mL 3M ammonium acetate is added and the plasmid DNA precipitated with l8 mL of ethanol. After chilling at ~20°C the DNA is pelleted by centrifugation at 3400Xg for 30 minutes. A second precipitation is similarly performed, then the DNA is rinsed with ethanol, vacuum dired, dissolved in l mL TE and the DNA concentration is determined spectrophotometrically.

## EXAMPLE 6: INTRODUCTION OF PLASMID LIBRARY INTO STREPTOMYCES LIVIDANS AND CONSTRUCTION OF A S. LIVIDANS RECOMBINANT CELL LIBRARY

The bifunctional plasmid library constructed in the previous step is transformed into Streptomyces lividans TK54 where phenotypic expression of Streptomyces genes is achieved. To this end protoplasts of Streptomyces lividans TK54 are prepared by essentially standard methods (Hopwood et al, l985). Briefly, the cells from a 45-hour culture of S. lividans TK54, developed by inoculating 0.2 mL of a spore suspension into each of ten-50 mL aliquots of complete YEME medium (3 g of yeast extract/L, 5 g of peptone/L, 3 g of malt extract/L, 10 g of glucose/L, 340 g of sucrose/L, 5 g of glycine/L, 5 mM MgCl$_2$, 40 mg/L each L-histidine and L-leucine) are pelleted by centrifugation at 9800Xg for l5 minutes. The cell pellet is washed twice with P medium and then suspended in 60 mL P$^+$. Twenty mL of P$^+$ containing l4 mg of lysozyme/mL is added and the suspension incubated at 30°C in a shaking water bath at 150 rpm for 90 minutes. Subsequently, l00 mL P$^+$ is added and the protoplast suspension is passed through sterile non-absorbent cotton. The filtrate is spun at 3800Xg for l0 minutes, the protoplast pellet resuspended and washed with l00 mL P+, and after a second centrifugation resuspended in l20 mL P$^+$. The protoplast preparation is distributed to l.8 mL cryotubes and frozen at -70°C. Transformation is conducted by distributing 0.3 mL of the TK54 protoplast preparation (containing ~1 Xl0$^9$ protoplasts) to each of 4 centrifuge tubes containing 5 mL P$^+$. The protoplasts are pelleted by spinning at 3400Xg for l0 minutes and then resuspended in the residual volume. Approximately l0 $\mu$g of cosmid library DNA is added to each, followed by the addition of 0.5 mL of 25% PEGI000 (I g PEGI000 [Sigma] dissolved in 3mL of a solution consisting of 25 g of sucrose/L, 2 mL 500X trace elements solution/L, 0.25 g of $K_2SO_4$/L, I00 mM $CaCl_2$, 50 mM TRIS-maleate pH 8). After mixing and incubating for 30 seconds, 5 mL P$^+$ is added. The protoplasts are then pelleted and resuspended in I mL P$^+$. One tenth ml volumes are then spread onto dried $R_2$YE agar (I00 g of sucrose/L 0.25 g of $K_2SO_4$/L, 2ml of 500X trace elements solution/L, 2g of L-proline IL, 20 g of D glucose/L, 5 g of yeast extract/L, 0.05 g of $KH_2PO_4$/L, 25 mM TES, 25 mM $CaCl_2$, 5 mM $MgCl_2$, 20 g of Bacto-agar/L) and incubated at 28°C. At 24 hours each plate is overlayed with 3 mL of soft R agar (formulated as above but without yeast extract, glucose, or $KH_2PO_4$ and containing 8g of Bacto-agon IL) containing 500 $\mu$g of thiostrepton/mL and then incubated an additional 12 days.

Approximately 9100 thiostrepton-resistant colonies are obtained. These are collected by scraping colonies from the agar plates into 3 tubes containing 25 mL each 20% glycerol. The colony suspensions are fragmented by sonicating for 90 seconds, pooled, then distributed to I.8 mL cryotubes and frozen at -70°C. This frozen preparation constitutes the S. lividans recombinant cell library.

EXAMPLE 7: ISOLATION OF S. LIVIDANS LL535. A TETRACYCLINE-RESISTANT TRANSFORMANT FROM WHICH PLASMID LP$^2$I27 IS DERIVED

The recombinant cell library of S. lividans is next subjected to a screen for tetracycline resistance. One-tenth mL portions of the fragmented S. lividans cell library are plated onto Bennetts agar supplemented with I00 $\mu$g of tetracycline/mL. After incubation at 28°C for 5 days, two tetracycline-resistant colonies are detected. One of these, LL535 (initially designated LL529-2) is chosen for further analysis. The LL535 colony is streaked to fresh Bennetts agar containing I00 $\mu$g of tetracycline/mL. Growth is observed after 3 days incubation at 28°C. The growth obtained is scraped into 50 mL TSB supplemented with I0 g of glucose/L (TSBG) and I00 $\mu$g of tetracycline/mL and the suspension incubated at 30°C, 200 rpm for 3 days. The LL535 culture is briefly sonicated and a portion distributed to I.8 mL cryotubes and stored at -70°C. The remaining volume is used to inoculate four 2 liter flasks containing 500 mL each modified YEME medium (as previously described but containing I6 g of glycine/L, 25 mM MOPS and without $MgCl_2$, L-histidine or L-leucine) containing I00 $\mu$g of tetracycline/mL. The growth obtained after two days is then processed for isolation of plasmid DNA as previously described except that all volumes employed are four times that of the previous example. The final DNA precipitate is dissolved in I mL TE.

A I0 $\mu$L portion of the plasmid DNA isolated from S. lividans transformant LL535 is subjected to an in vitro packaging reaction and subsequently transduced to E. coli X2819T using methods described hereinabove. An ampicillin-resistant transductant (designated LL537 is streaked to 20-10-5 agar containing I00 $\mu$g of ampicillin/mL and the growth obtained after a I-day incubation at 30°C is used to inoculate two 500 mL portions of 20-10-5 broth containing I00 $\mu$g of amplicillin/mL. After incubation at 30°C, 200 rpm overnight plasmid DNA is isolated again as previously described. The isolated plasmid is designated LP$^2$127; the estimated size of the plasmid is 43 kilobase pairs.

A restriction map is generated for LP$^2$I27 by performing single and double digests with restriction endonucleases. The location of cleavage sites for BamHI, BclI, BglIII, BsmI, BstBI, ClaI, EcoRI, MluI, NcoI, SacI, ScaI, SphI and StuI (New England Biolabs) are determined by digesting with each enzyme alone and in combination with enzymes that cut at known locations within the vector portion, such as EcoRI, EcoRV or HindIII. Restriction endonuclease digestions are performed by combining 1-2 $\mu$g of plasmid DNA 4 $\mu$l of a 10X solution of salts that are optimal for the restriction endonuclease being employed and approximately 5-40 units of enzyme in a total volume of 40 $\mu$l. The 10X salt solutions employed are as follows: for BamHI, EcoRV and SalI, 1.5M NaCl, 0.06M Tris PH8, 0.06M $MgCl_2$; for BglIII and ScaI, 1.0M NaCL, 0.IM Tris pH7.4, 0.IM $MgCl_2$; for BclI, 0.75M KCl, 0.06M Trish pH7.4, o.IM $MgCl_2$; for BstBI, 0.6M NaCl, 0.06M Tris pH 7.4, 0.06M $MgCl_2$; for ClaI, 0.5M NaCl, 0.06M Tris pH8, 0.06M Tris pH7.4, 0.06M $MgCl_2$; for ClaI, 0.5M NaCl, 0.06M Tris pH8, 0.06M $MgCl_2$; for EcoRi, 0.5M Tris pH8, 0.1M $MgCl_2$; for MluI, 0.5M$^2$NaCl, 0.IM Tris pH7.4, 0.IM $MgCl_2$; for SacI, 0.IM Tris pH7.4, 0.IM $MgCl_2$; and for StuI, I.0M NaCl, 0.IM Tris pH8, 0.IM Tris pH7.4, 0.IM $MgCl_2$; and for StuI, I.0M NaCl, 0.IM Tris pH8, 0.1M $MgCl_2$. Double digests are performed with salt conditions compatible for both enzymes, as recommended by the manufacturer. All digestion reactions are conducted at 37°C except for BclI which is performed at 50°C and BsmI and BstBI which are performed at 65°C. The incubation time is 60-120 minutes. A 5 $\mu$l volume of tracking dye (50% glycerol, 0.IM EDTA pH8, 0.25% bromphenol blue) is added to stop the reaction and to facilitate the subsequent loading of agarose gels.

Digestion results are visualized by electrophoresis through 0.8% agarose gels. The map is assembled by direct digestion of LP$^2$I27 as well as by digestion of subcloned fragments. Mapping for BclI and ClaI sites is inhibited by host methylation and, therefore, aided by the use of LP$^2$258, which is obtained by in vitro packaging of LP$^2$127, transduction to E. coli GM119 (dam-dcm-) and plasmid isolation by previously described procedures. The physical structure of LP$^2$127 is shown in Figure 2. A more detailed restriction endonuclease map for the 31.9kb at S. aureofaciens DNA cloned in LP$^2$127 is shown in Figure 3.

EXAMPLE 8: ISOLATION OF S. LIVIDANS LL529-TT2. THIOSTREPTON-RESISTANT, TETRACYCLINE-RESISTANT TRANSFORMANT-FROM WHICH PLASMID LP$^2$128 IS DERIVED

The isolation of Streptomyces lividans LL529-TT2 is performed in a similar fashion to that described for LL535 except that the recombinant S. lividans cell library is plated onto Bennetts agar containing 50 μg of thiostrepton/mL and l00 μg of tetracycline/mL. After incubation at 28ºC, for ll days, two resistant colonies are observed. One of these, LL529-TT2 is streaked to Bennetts agar containing both antibiotics. After three days incubation at 28ºC, the resulting growth is used to inoculate 50 mL TSB containing l0 μg of thiostrepton/mL and l00 μg of tetracycline/mL. After five days incubation at 28ºC, 200 rpm, plasmid DNA is prepared by a minipreparation procedure, which is similar to previously described plasmid isolation procedures up to the isopropanol precipitation step. However, in this case the volumes employed are ~1/4 of those previously noted. After isopropanol precipitation, the nucleic acid pellet is dissolved in l mL TE and extracted with an equal volume of chlorpane (500 g phenol and 0.5 g 8-hydroxyquinoline equilibrated in a buffer containing l00 mM NaCl, l mM EDTA pH8, l0 mM sodium acetate pH 6, plus 500 mL chloroform) by agitating and then spinning in a microfuge at full speed for 3 minutes. The aqueous phase is next re-extracted with chlorpane and then extracted with chloroform in a similar fashion. The final aqueous layer is collected and l00 μL 3M ammonium acetate and l.8 mL ethanol are added to precipitate nucleic acids. After chilling to -20ºC, the precipitation reaction is centrifuged at 8800Xg for 30 minutes. The resulting pellet is dissolved in 300 μL 0.3M ammonium acetate, similarly precipitated with l mL ethanol and centrifuged. The resulting pellet is rinsed with ethanol, vacuum dried and dissolved in l mL TE.

The LL529-TT2 plasmid minipreparation is used to perform in vitro packaging as previously described. An ampicillin-resistant transductant (designated LL538) is chosen for plasmid preparation which is performed as outlined in the example for LL535. The resulting purified plasmid is designated LP$^2$l28. The restriction band patterns obtained when LP$^2$l28 is digested with 20 different restriction endonucleases is compared to that obtained for LP$^2$l27 by analyzing the digestion products on the same electrophoresed agarose gel. The gel banding patterns obtained for LP$^2$l28 are identical to those seen for LP$^2$l27, indicating that the two plasmids are equivalent. Thus, Figures 2 and 3 describe the structure of LP$^2$l28 as well as LP$^2$l27.

EXAMPLE 9: PLASMID LP$^2$127 AND LP$^2$128 CONFER A PLASMID LINKED TETRACYCLINE RESISTANCE

To verify that the tetracycline resistances encountered are plasmid-borne, plasmids LP$^2$l27 and LP$^2$l28 are transformed into protoplasts of Streptomyces lividans and the resulting thiostrepton-resistant transformants are tested for tetracycline-resistance. The procedures for preparation and transformation of S. lividans protoplasts and the subsequent selection for thiostrepton-resistant transformants are performed as previously described. Ten μg of both LP$^2$l27 and LP$^2$l28 are transformed, as well as 5 μg of a tetracycline-sensitive control, LP$^2$lll (a vector consisting of pIBl24, the SCP2[*] replication and stability regions and thiostrepton-resistance gene). One hundred and fifty transformants for each plasmid tested are sequentially picked to pairs of Bennetts' agar plates containing l00 μg of tetracycline/mL or 25 μg of thiostrepton/mL.

Growth of the stabs are scored after incubation at 28ºC for 5 days. All the thiostrepton-resistant transformants derived from LP$^2$lll prove to be tetracycline-sensitive, whereas 80% of thiostrepton-resistant transformants tested from either LP$^2$l27 or LP$^2$128 are shown to be tetracycline-resistant.

EXAMPLE 10: PRODUCTION OF CHLORTETRACYCLINE AND TETRACYCLINE BY STREPTOMYCES LIVIDANS CONTAINING LP$^2$127 AND LP$^2$128

A series of experiments is performed to demonstrate that LP$^2$l27 and LP$^2$l28 direct the biosynthesis of chlortetracycline (CTC) and tetracycline (TC) in the heterologous host Stretomyces lividans. The original isolate LL535, as well as S. lividans transformed with LP$^2$l27, produce CTC and TC on agar and in broth fermentation, whereas S. lividans containing a plasmid cloning vector without inserted DNA does not yield a tetracycline antibiotic. LP$^2$l28 transformed into S. lividans directs the synthesis of an antiobiotic with acitivity against Escherichia coli that can be biologically characterized as tetracycline. No such activity is produced by the S. lividans host.

Initially, S. lividans strain LL535, the thiostrepton-resistant isolate from which LP$^2$l27 is isolated, is plated onto Bennetts agar (which contains 25 $\mu$g of thiostrepton/mL) at a cell dilution designed to give approximately 200 colonies per plate. S. lividans strain LL531 (which contains the previously described plasmid vector LP$^2$lll) is similarly plated at a target density of ~400 colonies per plate.

After eight days incubation at 30$^o$C, colonies of LL535 exhibit a yellow UV flourescence when illuminated with a 366 nm UV lamp. This is characteristic of tetracycline producing cultures and is not observed for LL531.

Plates of each then are tested for biological activity by overlaying the colonies with 5 mL soft 20-10-5 agar (8 g of Bacto agar/L) which has been seeded with 0.l mL of an overnight growth of assay organism. The assay strains employed are Bacillus subtilis strain Tl325 obtained from the University of Leicester (Dr. Eric Clundliffe) Tl325 (which contains a plasmid conferring thiostrepton-resistance), Escherichia coli MM294 and MM294 (ATCC 33625) containing pBR322 (which confers tetracycline and ampicillin resistance). The overnights are developed at 37$^o$C in l0 mL 20-10-5, which is supplemented with 25 $\mu$g of thiostrepton/mL for Tl325 and l00 $\mu$g of ampicillin/mL for MM294/pBR322. Once overlayed and incubated at 37$^o$C overnight, the plates are examined for zones of inhibition in the lawns of overlay organism. Strain LL531 gives no zones with E. coli strains and only a few colonies give small localized zones with the gram-positive Tl325. All of these latter colonies show red-pigment which is characteristic of expression of actinorhodin; S. lividans is known to express this normally cryptic pathway at observable frequency (Horinouchi et al, l989). By comparison, strain LL535 shows production of an antibiotic that totally inhibits growth of the Tl325 and MM294 in the overlay (In later experiments with fewer colonies per plate, small, discrete and very large zones of inhibition are seen around suitably separated individual colonies with these assay organisms). Colonies of LL535 overlayed in the present experiment with MM294/pBR322 show discrete zones around colonies. This reduced activity effect seen with MM294/pBR322 is taken to indicate a reduced sensitivity owing to the expression of tetracycline resistance resident on plasmid pBR322.

The antibiotic being elaborated on agar is characterized by extracting antibiotic from agar blocks of confluent plate cultures. Strains LL535 and LL531 are grown on Bennetts agar containing 25 $\mu$g of thiostrepton/mL; S. aureofaciens ATCC l3899, the source of the cloned DNA in LP$^2$l27 and LP$^2$l28, is plated on Bennetts agar without drug. After five days of growth at 30$^o$C, l" square agar blocks are cut out and macerated in 3 mL acid methanol (ll.5

mL concentrated $H_2SO_4$ in 4 liters methanol). After vortexing for five minutes, the supernatant is filtered through an Acro®LC-25 membrane filter and subjected to HPLC analysis.

The HPLC analyses are carried out isocractically on a Cl8 reverse phase column with a mobile phase consisting of oxalate buffer at pH 2.9 containing 22% DMF = N.N-dimethylformamide. Flowrate is l mL/min and the eluate is monitored at 365 nm. Authentic tetracycline and chlortetracycline are used as standards.

The HPLC chromatograms show that LL535 and ATCC l3899 are producing substances with retention times indentical to TC and CTC. Extracts of LL531 do not show these peaks.

Thiostrepton-resistant transformants of S. lividans obtained with LP$^2$l27, LP$^2$l28 and LP$^2$63 (a plasmid vector consisting of pIBl24 cloned into the SacI site of pIJ702) are similarly analyzed for production of antibiotic by overlay with assay organisms Tl325 and MM294. The LP$^2$l27 and LP$^2$l28 transformants show production of antibiotic(s) active against both assay organisms whereas LP$^2$63 transformants do not, thereby indicating that the ability to produce antibiotic is associated with the S. aureofaciens DNA present in LP$^2$l27 and LP$^2$l28.

Broth fermentations are also conducted as an additional confirmation that the antibiotics being produced by S. lividans bearing LP$^2$l27 are tetracycline and chlortetracycline. Fifty mL seed cultures of ATCC l3899, LL531, and LL873 (a LP$^2$l27 transformant of S. lividans) are developed using S medium (4 g of yeast extract/L, 4 g of peptone/L, l0 g of glucose/L, 0.5 g of $MgSO_4 \cdot 7H_2O$/L) containing 5 $\mu$g of thiostrepton/mL; ATCC l3899 was grown without thiostrepton. After incubation at 30$^o$C for three days, 0.5 mL seed is transferred to 25-mL fermentations containing l0 $\mu$g of thiostrepton/mL (except no drug with ATCC l3899). After incubation at 28$^o$C for ten days, 0.5 mL samples of the final mashes are diluted into 4.5 mL acid methanol, processed as previously described and subjected to HPLC analysis. Strain LL531 yields no tetracycline compounds, whereas ATCC l3899, LL535 and LL873 yield 37, 56 and 6 $\mu$g/mL CTC respectively. A small amount of TC also is detected in the fermentation mashes of these three strains.

E. coli strains LL537 and LL538 are the E. coli transductants from which plasmids LP$^2$l27 and LP$^2$l28 are isolated and have been deposited, under the Budapest Treaty, in the American Type Cell Culture, 12301 Parklawn Drive, Rockville, Maryland and have ATCC accession numbers as follows. E. coli X2818T containing LP$^2$l27 (LL537) has accession number ATCC 68357, and E. coli X2819T containing LP$^2$l28 (LL538) has accession

number ATCC 68358. Both were filed on July 10, 1990 and are available to the public when legally applicable.

BIBLIOGRAPHY

1. Baltz, R.H. and P. Matsushima. l98l. Protoplast fusion in Streptomyces: conditions for efficient genetic recombination and cell regeneration. J. Gen. Microbiol. l27:l37-146.

2. Baltz, R.H. and E.T. Seno. l988. Genetics of Streptomyces fradiae and tylosin biosnthesis. Ann. Rive. Microbiol. 42:547-574.

3. Bibb, M.J., J.M. Ward, and D.A. Hopwood. l978. Transformation of plasmid DNA into Streptomyces protoplasts at high frequency. Nature (London) 274:398-400.

4. Binnie, C., M. Warren, and M.J. Butler. l989. Cloning and heterologous expression in Streptomyces lividans of Streptomyces rimosus genes involved in oxytetracycline biosynthesis. J. Bateriol. l7l:887-895.

5. Butler, M.J., E.J. Friend, I.S. Hunter, F.S. Kaczmarek, D. A. Sugden and M. Warren. l989. Molecular cloning of resistance genes and architecture of a linked gene cluster involved in biosynthesis of oxytetracycline by Streptomyces rimosus. Mol. Gen. Genet. 215:231-238.

6. Chater, K.F. and C.J. Bruton. l983. Mutational cloning in Streptomyces and the isolation of antibiotic production genes. Gene 26:67-78

7. Chen, C.W., H.-F. Lin, C.L. Kuo. H.-L. Tsai and J.F.-Y. Tsai. l988. Cloning and expression of a DNA sequence conferring cephamycin C production. Bio/Technology 6:l222-1224.

8. Cox, K.L. and R.H. Baltz. l984. Restriction of bacteriophage plaque formation in Streptomyces spp. J. Bacteriol. l59:499-504.

9. Distler, J., K. Mansouri and W. Piepersberg. l985. Streptomycin biosynthesis in Streptomyces griseus II. Adjacent genomic location of biosynethetic genes and one of two stretomycin resistance genes. FEMS Microbiol. Lett 30:151-154.

10. Duggar, B.M. l948. Aureomycin: a product of the continuing search for new antibotics. Ann. N.Y. Acad. Sci. 51:l7l-l8l.

11. Feitelson, J.S. and D.A. Hopwood. l983. Cloning of a Streptomyces gene for an O-methyltransferase involved in antibiotic biosynthesis. Mol. Gen. Genet. l90:394-398.

12. Fishman, S.E., K. Cox, J.L. Larson, P.A. Reynolds, E.T. Seno, W.-K Yeh, R. Van Frank and C.L. Hershberger. l987. Cloning genes for the biosynthesis of a macrolide antibiotic. Proc. Natl. Acad. Sci. USA 84:8248-8252.

13. Gil, J.A. and D.A. Hopwood. l983. Cloning and expression of a p-aminobenzoic, acid synthetase gene of the candicidin-producing Streptomyces griseus. Gene 25:ll9-132.

14. Goodman, J.J. l985. Fermentation and mutational development of the tetracyclines. p.5-57. In: J.J. Hlavka and J.H. Boothe (eds), Handbook of Experimental Pharmacology, vol. 78, The Tetracyclines. Springer-Verlag, Berlin.

15. Hohn, B. and J. Collins. A small cosmid for efficient cloning of large DNA fragments. Gene 11:291-298.

16. Hopwood, D.A. and H.M. Wright. l978. Bacterial protoplast fusion: recombination in fused protoplasts of Streptomyces coelicolor. Mol. Gen. Genet. l62:307-317.

17. Hopwood, D.A., l967. Genetic analysis and genome structure in Streptomyces coelicolor. Bacteriol. Rev. 31:373-403.

18. Hopwood, D.A., M.J. Bibb, K.F. Chater, T. Kieser, C.J. Bruton, H.M. Kieser, D.J. Lydiate, C.P. Smith, J.M. Ward and H. Schrempf. l985. Genetic manipulation of Streptomyces- a laboratory manual. The John Innes Foundation. Norwich, England.

19. Horinouchi, S., F. Malpartida, D.A. Hopwood and T. Beppu. l989. afsB stimulates transcription of the actinorhodin biosynthetic pathway in Streptomyces coelicolor A3 (2) and Streptomyces lividans. Mol. Gen. Genet. 215:355-357.

20. Jones, G.H. and D.A. Hopwood. l984. Molecular cloning and expression of the phenoxazinone synthase gene from Streptomyces antibioticus. J. Biol. Chem. 259:14151-14157.

21. Katz, E., C.J. Thompson and D.A. Hopwood. 1983. Cloning and expression of the tyrosinase gene from Streptomyces antibioticus in Streptomyces lividans. J. Gen. Microbiol. 129:2703-2714.

22. Larson, J.L. and C.L. Hershberger. 1986. The minimal replicon of a streptomycete plasmid produces ultrahigh level of plasmid DNA. Plasmid 15:199-209

23. Leskiw, B.K., Y. Aharonowitz, M. Mevarech. S. Wolfe, L.C. Vining. D.W.S. Westlake and S.E. Jensen. l988. Cloning and nucleotide sequence determination of the isopenicillin N synthetase gene from Streptomyces clavuligerus. Gene 62:l87-l96.

24. Lydiate, D.J., F. Malpartida and D.A. Hopwood. l985. The Streptomyces plasmid SCP2*: its funtional analysis and development into useful cloning vectors. Gene 35:223-235.

25. Malpartida, F. and D.A. Hopwood. l984. Molecular cloning of the whole biosynthetic pathway of a Streptomyces antibiotic and its expression in a heterologous host. Nature (London) 309:462-464.

26. Maniatas, T., E.F. Fritsch and J. Sambrook. l982. Molecular cloning: a laboratory manual.

Cold Spring Harbor Laboratory, Cold Spring Harbor, NY.

27. McCormick, J.R.D. l968. Point blocked mutants and biogenesis of tetracyclines. p. l63-173. In: G. Sermonti and M. Alecevic (eds), Genetics and Breeding of Streptomycetes. Yugoslav. Acad. Sci. and Arts, Zagreb.

28. Motamedi, H. and C.R. Hutchinson. l987. Cloning and heterologous expression of a gene cluster for the biosynthesis of tetracenomycin C, the anthracycline antitumor antbiotic of Streptomyces glaucescens. Proc. Natl. Acad. Sci. USA 84:4445-4449.

29. Murakami, T., H. Anzai, S. Imai, A. Satoh, K. Nagaoka and C.J. Thompson. l986. The bialaphos biosynthetic genes of Streptomyces hygroscopicus: molecular cloning and characterization of the gene cluster. Mol. Gen. Genet. 205:42-50.

30. Reynes, J.P., T. Calmels, D. Drocourt and G. Tiraby. 1988. Cloning, expression in Escherichia coli and nucleotide sequence of a tetracycline-resistance gene from Streptomyces rimosus. J. Gen. Microbiol. 134:585-598.

31. Sanger, F., A.R. Coulson, G.F. Hong, D.F. Hill and G.B. Peterson. 1982. Nucleotide sequence of bacteriophage lambda DNA. J. Mol. Biol. 162:729-773.

32. Stanzak, R., P. Matsushima, R.H. Baltz and R.N. Rao. l986. Cloning and expression in Streptomyces lividans of clustered erythromycin biosynthesis genes from Streptomyces erythreus. Bio/Technology 4:229-232.

33. Stutzman-Engwall, K.J. and C.R. Hutchinson. l989. Multigene families for anthracycline antibiotic production in Streptomyces peucetius. Proc. Natl. Acad. Sci. USA 86:3135-3139.

34. Sutcliffe, J.G. 1979. Complete nucleotide sequence of the Escherichia coli plasmid pBR322. Cold Spring Harbor Symp. Quant. Biol. 43:77-90.

35. Thompson, C.J., T. Kieser, J.M. Ward, and D.A. Hopwood. l982. Physical analysis of antibiotic-resistance genes from Streptomyces and their use in vector construction. Gene 20:51-62.

36. van Pee, K.-H. l988. Molecular cloning and high-level expression of a bromoperoxidase gene from Streptomyces aureofaciens Tu24. J. Bacteriol. l70:5890-5894.

37. Vara, J.A., D. Pulido, R.A. Lacalle and A. Jimenez. l988. Two genes in Streptomyces alboniger puromycin biosynthesis pathway are closely linked. Gene 69:l35-l40.

38. Veselova, S.I. l969. Combined effect of nitrous acid, ultraviolet light, streptomycin and chlortetracycline on Actinomyces aureofaciens. Antiobiotiki l4:698-702.

**Claims**

1. The isolated DNA gene cluster coding for the biosynthetic pathway of tetracycline and chlortetracycline.

2. The DNA according to Claim l, wherein said DNA under stringent hybridization conditions hybrizes to the DNA of Claim 1.

3. The DNA according to Claim 2, wherein said DNA is expressed in a prokaryotic host.

4. The DNA according to Claim 3, wherein said host is E. Coli, Streptomyces lividans, Streptomyces griseofuscus, Streptomyces ambofuchsus, Actinomycetes, Bacillus, Cornebacteria, or Thermoactinomyces.

5. The DNA according to Claim 4, wherein said host is Streptomyces lividans.

6. The DNA according to Claim 5, wherein said DNA is the DNA gene cluster located in LP$^2$127 or LP$^2$128.

7. A method for enhancing the yield of tetracycline, chlortetracycline or analogues thereof, said method comprising: regulating the biosynthetic pathway of a microorganism producing said antibiotics by manipulating the DNA gene cluster encoding said biosynthetic pathway to enhance production of tetracycline, chlortetracycline or analogues thereof.

8. A method according to Claim 7, wherein said DNA is expressed in a prokaryotic host.

9. A method to Claim 8, wherein said host is E. coli, Streptomyces lividans, Streptomyces griseofuscus, Streptomyces ambofuchsus, Actinomycetes, Bacillus, Cornebacteria, or Thermoactinomyces.

10. A method according to Claim 9, wherein said host is Streptomyces. lividans.

11. A method according to Claim 10, wherein said DNA the DNA gene cluster is located in LP$^2$127 or LP$^2$128.

12. A method for screening for antibiotic analogues of tetracycline or clortetracycline, said method comprising: screening for resistance transformants containing the DNA gene cluster encoding the biosynthetic pathway or portion thereof for production of tetracycline or chlortetracycline.

**13.** A method according to Claim 12, wherein said DNA under stringent hybridization conditions hybridizes to the DNA of Claim 12.

**14.** A method according to Claim 13, wherein said DNA is expressed in a prokaryotic host.

**15.** A method according to Claim 14, wherein said DNA is the DNA gene cluster located in $LP^2127$ or $LP^2128$.

FIG I

FIG II

# FIG    III

| | | | | | | | | | |
|---|---|---|---|---|---|---|---|---|---|
| 1.0 | 6.9 | 11.8 | | 2.6 | 0.4 | 5.9 | 1.2 | 0.7 1.4 | **Bam HI** |
| 16.8 | | | 4.7 | | | 10.4 | | | **Bcl I** |
| 1.7 | 22.5 | | | 1.3 | | 6.4 | | | **Bgl II** |
| 4.2 | 27.7 | | | | | | | | **Bam I** |
| 9.4 | | 22.5 | | | | | | | **Bst BI** |
| 3.0 | 6.6 | 22.3 | | | | | | | **Cla I** |
| 1.8 | 20.1 | | | | 10.0 | | | | **EcoR I** |
| 28.1 | | | | | | | 3.8 | | **Mlu I** |
| 2.7 | 4.3 | 0.7 | 5.7 | 1.6 | 9.6 | | 5.5 | 1.8 | **Nco I** |
| 2.7 | 0.6 0.7 | 3.8 | 3.8 | 3.2 | 2.0 | 2.3 | 1.6 | 11.2 | **Sac I** |
| 4.5 | 13.7 | | | 0.5 | 7.4 | | 5.8 | | **Sca I** |
| 2.5 | 2.5 | 9.9 | | 1.0 | 2.5 | 6.9 | | 6.6 | **Sph I** |
| 7.8 | 11.2 | | | 2.6 | 6.4 | | 2.4 | 1.5 | **Stu I** |

Kbs

5    10    15    20    25    30  31.9

EP 0 468 220 A2